# EUROPÄISCHE PATENTANMELDUNG

(11) **EP 2 816 095 A1**
(43) Veröffentlichungstag der Anmeldung: **24.12.2014**
(21) Anmeldenummer: 13172270.4
(22) Anmeldetag: 17.06.2013
(51) Int. Cl.: C10J 3/72, B07C 5/344, B07C 5/346, G01N 23/00, G01N 23/223, G01N 1/04

(54) **Verbesserte Brennstoffvergasung**

(71) Anmelder: Siemens Aktiengesellschaft, 80333 München (DE)
(72) Erfinder: Kiener, Christoph, Dr., 81369 München (DE)

(57) **Zusammenfassung**

Die Erfindung führt ein Verfahren zum Betreiben einer Brennstoffvergasungsvorrichtung (1) ein, das wenigstens die folgenden Schritte umfasst:
Zuführen von kohlenstoffbasiertem Brennstoff zu der Brennstoffvergasungsvorrichtung (1);
Zuführen eines Vergasungsmittels zu der Brennstoffvergasungsvorrichtung (1);
Erhitzen des Brennstoffs und des Vergasungsmittels in der Brennstoffvergasungsvorrichtung (1) in einer sauerstoffarmen Atmosphäre für die Produktion eines Synthesegases.

Erfindungsgemäß wird dabei der zugeführte Brennstoff von einer Analysevorrichtung (4, 14) untersucht und ein jeweiliger Gehalt einer Mehrzahl von Begleitelementen an dem Brennstoff bestimmt. Die Brennstoffvergasungsvorrichtung (1) wird in Abhängigkeit von den bestimmten Gehalten der Mehrzahl von Begleitelementen betrieben.

Die Erfindung betrifft außerdem eine Brennstoffvergasungsvorrichtung (1) und die Verwendung einer solchen zur Herstellung eines Synthesegases.

## Beschreibung

Die Erfindung betrifft ein Verfahren zum Betreiben einer Brennstoffvergasungsvorrichtung und eine Brennstoffvergasungsvorrichtung, die nach dem erfindungsgemäßen Verfahren betrieben wird.

Bei der Vergasung von Brennstoffen wird ein fester kohlenstoffbasierter Brennstoff in ein Synthesegas umgewandelt, das in chemischen Prozessen als Ausgangsstoff Verwendung finden kann. Dazu wird der Brennstoff in einer sauerstoffarmen Atmosphäre unter Zuführung eines Vergasungsmittels (beispielsweise reiner Sauerstoff mit Wasser und/oder Kohlenstoffdioxid) stark erhitzt, wodurch eine autotherme chemische Reaktion zwischen dem Kohlenstoff des Brennstoffs und dem Vergasungsmittel bewirkt wird. Das Synthesegas enthält dementsprechend insbesondere Kohlenmonoxid und Wasserstoff.

Für die autotherme Vergasung wird der Brennstoff auf eine Temperatur von bis zu 2500 Grad Celsius erhitzt. Die hierfür benötigte Energie wird durch eine teilweise Verbrennung des Brennstoffs erzeugt, die anhand einer üblicherweise mit dem Vergasungsmittel zugeführten Sauerstoffmenge kontrolliert wird. Dabei ist es aus Gründen der Wirtschaftlichkeit wünschenswert, einen möglichst geringen Anteil des Brennstoffs derartig zu verbrauchen, da andernfalls ein entsprechend geringerer Anteil des Brennstoffs in das Synthesegas umgewandelt werden kann. Gleichzeitig wird durch ein effizienteres Vergasungsverfahren der Ausstoß an Kohlendioxid gesenkt und der Aufwand für die Sauergaswäsche, der das Synthesegas unterzogen wird, gesenkt. Zudem wird weniger gasförmiger Sauerstoff benötigt, was ebenfalls eine Senkung der Verfahrenskosten bedingt.

Als Brennstoffe kommen beispielsweise Kohle, Petrolkoks, Teeröle, Raffinerierückstände oder auch Biomasse wie Torf, Holzpellets, Stroh, torrefizierte Biomasse, Biomasseöle aus Flash-Pyrolyse und dergleichen infrage. Im Rahmen der Erfindung wird der kohlenstoffhaltige Ausgangsstoff als "Brennstoff" bezeichnet, obgleich er bei der Vergasung nicht im eigentlichen Sinn verbrannt wird. Jeder Brennstoff beinhaltet jedoch neben Kohlenstoff eine Vielzahl von chemischen Begleitelementen in Form von Verunreinigungen, die eine Vielzahl von teilweise unbekannten chemischen Reaktionen eingehen. Aufgrund dieser Verunreinigungen entsteht bei dem Vergasungsprozess Asche. Die bei der Vergasung herrschende Temperatur sollte hoch genug sein, um die Asche zu einem Großteil zu flüssiger Schlacke zu verschmelzen, da die aschebildenden Bestandteile andernfalls als Feinstaub dem Synthesegas beigemischt sind und aufwendig abgetrennt werden muss.

Die Erfindung macht es sich zur Aufgabe, ein verbessertes Verfahren zum Betreiben einer Brennstoffvergasungsvorrichtung einzuführen.

Die Aufgabe wird durch das Verfahren des Anspruchs 1 gelöst. Die Erfindung führt ein Verfahren zum Betreiben einer Brennstoffvergasungsvorrichtung ein, das wenigstens die folgenden Schritte umfasst:
Zuführen von kohlenstoffbasiertem Brennstoff zu der Brennstoffvergasungsvorrichtung;
Zuführen von Vergasungsmittel (beispielsweise reiner Sauerstoff und Wasser und / oder Kohlenstoffdioxid) zu der Brennstoffvergasungsvorrichtung;
Erhitzen des Brennstoffs und des Vergasungsmittels in der Brennstoffvergasungsvorrichtung in einer sauerstoffarmen Atmosphäre für die Produktion eines Synthesegases.

Erfindungsgemäß wird dabei der zugeführte Brennstoff von einer Analysevorrichtung untersucht und ein jeweiliger Gehalt einer Mehrzahl von Begleitelementen an dem Brennstoff bestimmt. Die Brennstoffvergasungsvorrichtung wird in Abhängigkeit von den bestimmten Gehalten der Mehrzahl von Begleitelementen betrieben. Als "Begleitelemente" werden hier Elemente bezeichnet, die in variablen Anteilen neben Kohlenstoff in dem Brennstoff enthalten sind. Je nach Vergasungsverfahren können die Summe aller Begleitelemente oder auch einzelne Begleitelemente bis zu 50 Gewichtsprozent des Brennstoffs darstellen.

Die Untersuchung durch die Analysevorrichtung wird je nach Ausführung der Analysevorrichtung entweder kontinuierlich oder in regelmäßigen Abständen durchgeführt. Eine häufigere Untersuchung wird dabei eine genauere Abstimmung der Prozessparameter erlauben, bringt jedoch entsprechend höheren Aufwand mit sich. Es können zudem zusätzliche Untersuchungen vorgenommen werden, wenn eine Änderung des zugeführten Brennstoffs beispielsweise durch Umstellung auf einen anderen Brennstoff oder durch Entnahme von Brennstoff aus einer anderen Liefercharge, einer anderen Lagerstätte oder einem anderen Lager zu erwarten ist.

Die Untersuchungen können entweder an der Gesamtheit des zugeführten Brennstoffs oder an aus dem zugeführten Brennstoff automatisiert entnommenen Proben durchgeführt werden.

Die Erfindung bietet den Vorteil, dass die genaue chemische Zusammensetzung des zugeführten Brennstoffs bekannt ist, wodurch das Vergasungsverfahren und gegebenenfalls die der Brennstoffvergasungsvorrichtung nachgeschalteten Prozessanlagen wie die Gaswäsche in vorteilhafter Weise gesteuert werden können.

Beispielsweise kann die Zuführung des Brennstoffs gestoppt werden, wenn eine Überschreitung eines Grenzwertes durch den Gehalt eines bestimmten Begleitelementes durch die Analysevorrichtung festgestellt wird. Dadurch kann eine Beeinträchtigung der Brennstoffvergasungsvorrichtung durch aggressive Stoffe oder deren Reaktionsprodukte verhindert werden. Beispielsweise ist von manchen Stoffen bekannt, dass sie einen bei der Vergasung oder der Nachbehandlung des erzeugten Synthesegases verwendeten Katalysator kontaminieren und damit unwirksam werden lassen. Zudem kann ein unerwünscht hoher Ausstoß von umweltschädigenden Stoffen vermieden werden, wenn bekannt ist, dass das oder die bestimmten Begleitelemente in der Brennstoffvergasungsvorrichtung zu einer Erzeugung dieser umweltschädigenden Stoffe führen.

Die von der Analysevorrichtung bestimmten jeweiligen Gehalte der Begleitelemente können für einen Betriebszyklus der Brennstoffvergasungsvorrichtung rechnerisch akkumuliert werden. Die Brennstoffvergasungsvorrichtung kann dann in Abhängigkeit der akkumulierten bestimmten Gehalte der Begleitelemente betrieben werden. Diese Ausführungsform der Erfindung erlaubt die Bestimmung einer Ablagerung von spezifischen Verunreinigungen in der Brennstoffvergasungsvorrichtung beziehungsweise in den der Brennstoffvergasungsvorrichtung nachgeschalteten Prozessanlagen und der genauen Zusammensetzung der Ablagerungen, wodurch eine Steuerung des Brennstoffvergasungsverfahrens unter Berücksichtigung der Einflüsse der Ablagerungen auf die Brennstoffvergasung erfolgen kann. Solche spezifischen Verunreinigungen können Schadstoffe wie Quecksilber, Arsen, Selen oder Blei sein, unter Umständen in jeweils ganz spezifischen Verbindungen gesondert erfasst (beispielsweise AsH₃, elementares Quecksilber oder Blei, H₂Se). Dabei ist es auch denkbar, die verschiedenen Gehalte der Begleitelemente durch ein rechnerisches Modell zu bestimmen, bei dem ein Abtransport der Begleitelemente in unterschiedlichen chemischen Verbindungen aus der Brennstoffvergasungsvorrichtung durch geschmolzene Schlacke, Staubpartikel oder als flüchtige Verbindung mit dem Synthesegas berücksichtigt wird. Der Abtransport kann dabei für jedes Begleitelement anhand der Betriebsparameter wie Temperatur, Druck, Massenströme und Füllstand der Brennstoffvergasungsvorrichtung unter Berücksichtigung des chemischen und physikalischen Verhaltens des jeweiligen Begleitelementes berechnet werden.

Der Betriebszyklus der Brennstoffvergasungsvorrichtung kann beendet werden, wenn einer oder mehrere der akkumulierten bestimmten Gehalte einen Schwellwert überschreitet. Dadurch können die Zeitpunkte, zu denen eine Wartung, ein Austausch eines in der Brennstoffvergasungsvorrichtung verwendeten Katalysators oder eine Reinigung der Brennstoffvergasungsvorrichtung notwendig werden, genau bestimmt werden, so dass die Brennstoffvergasungsvorrichtung nur möglichst selten außer Betrieb gesetzt werden muss, was die Wirtschaftlichkeit des Betriebs erhöht.

Bei besonders bevorzugten Ausführungsformen der Erfindung wird wenigstens ein resultierender Gehalt der Mehrzahl von Begleitelementen angepasst und dadurch ein Schmelzpunkt einer während des Betreibens der Brennstoffvergasungsvorrichtung entstehenden Asche geändert. Die Erfindung macht sich dabei die Erkenntnis zunutze und schließt diese mit ein, dass die Zusammensetzung der Begleitelemente im Brennstoff und damit auch in der entstehenden Asche den Schmelzpunkt der Asche und das temperaturabhängige Fließverhalten der geschmolzenen Asche (Schlackeviskosität) wesentlich beeinflusst. Wird der Schmelzpunkt gesenkt, kann das Brennstoffvergasungsverfahren bei niedrigeren Temperaturen durchgeführt werden, was in der Praxis durch eine Verringerung der zugeführten Sauerstoffmenge erreicht wird. Dies bedingt jedoch auch eine Erhöhung der Effizienz des Verfahrens, weil ein geringerer Teil des Brennstoffs für dessen Erhitzung verbrannt werden muss. Dementsprechend sinkt auch der Ausstoß an Kohlendioxid und die Menge an Synthesegas wird erhöht. Eine gezielte Veränderung der Schlackeviskosität kann den Einsatz mancher Brennstoffe erst ermöglichen, da für die Durchführung des Brennstoffvergasungsverfahrens bestimmte Schlackeeigenschaften vorteilhaft sind. Daher kann es in Einzelfällen auch vorteilhaft sein, den resultierender Gehalt der Mehrzahl von Begleitelementen so anzupassen, dass dadurch der Schmelzpunkt der während des Betreibens der Brennstoffvergasungsvorrichtung entstehenden Asche erhöht wird, wenn dadurch eine vorteilhafte Schlackeviskosität erreicht wird.

Beispielsweise können die resultierenden Gehalte der Begleitelemente angepasst werden, indem das Zuführen des Brennstoffs ein Zuführen eines ersten Ausgangsbrennstoffs und eines von dem ersten Ausgangsbrennstoff verschiedenen zweiten Ausgangsbrennstoffs umfasst, indem das Bestimmen der Gehalte der Begleitelemente jeweils für den ersten Ausgangsbrennstoff und für den zweiten Ausgangsbrennstoff durchgeführt wird und indem ein Mischungsverhältnis von erstem und zweitem Ausgangsbrennstoff in Abhängigkeit der bestimmten Gehalte der Mehrzahl von Begleitelemente gewählt wird. Die Gehalte an Begleitelementen können also angepasst werden, indem zwei unterschiedliche Brennstoffe mit unterschiedlichen Gehalten an Begleitelementen in einem variablen Verhältnis zueinander gemischt werden. Dies können beispielsweise zwei unterschiedliche Arten von Kohle aus unterschiedlichen Abbaugebieten oder dergleichen sein. Es können selbstredend auch mehr als zwei verschiedene Brennstoffe miteinander gemischt werden, um die gewünschten resultierenden Gehalte an Begleitelementen zu erreichen.

Alternativ oder zusätzlich kann das Verfahren einen zusätzlichen Schritt des Zuführens von Zusatzstoffen zu der Brennstoffvergasungsvorrichtung in Abhängigkeit von den bestimmten Gehalten der Begleitelemente aufweisen. Hierbei werden die resultierenden Gehalte der Begleitelemente angepasst, indem Begleitelemente in einer von den bestimmten Gehalten abhängenden Menge zu dem Brennstoff hinzugefügt werden. Dies kann zusätzlich zu der oben beschriebenen Mischung von verschiedenen Brennstoffen geschehen.

Insbesondere können beispielsweise die resultierenden Gehalte von Calcium, Aluminium und Silicium angepasst werden, darüber hinausgehend auch von anderen oxidbildenden Metallen. Die Gehalte werden dabei bevorzugt als Oxide der Elemente berechnet, also als Gehalte von Calciumoxid (CaO), Aluminiumoxid (Al₂O₃) und Siliciumdioxid (SiO₂), also der Stoffe, die in der Asche zu erwarten sind. Im Brennstoff selbst können beispielsweise Calciumcarbonat oder Calciumalumosilikate enthalten sein, die entsprechend unterschiedlich zum Gehalt der Asche an CaO, Al₂O₃ und SiO₂ beitragen. Die Oxide werden daher als rechnerische Größe verwendet, um den Gehalt an Calcium, Aluminium und Silicium zu beschreiben. Die genannten Elemente stellen den größten Anteil an den in üblichen Brennstoffen aufgefundenen Begleitelementen und Verunreinigungen und haben dementsprechend einen besonders großen Einfluss auf die Schmelztemperatur der bei der Brennstoffvergasung entstehenden Asche. Günstige resultierende Gehalte an Begleitelementen - also solche Gehalte, die eine besonders niedrige Schmelztemperatur bewirken - können aus einem Phasendiagramm des Systems CaO-Al₂O₃-SiO₂ abgelesen werden. Die Erfindung erlaubt über die Steuerung der im Brennstoff enthaltenen Begleitelemente einen Betrieb im Eutektikum der bei der Brennstoffvergasung entstehenden Asche.

Vorzugsweise wird bei dem erfindungsgemäßen Verfahren ein Massenstrom eines zu der Brennstoffvergasungsvorrichtung zugeführten Sauerstoffstroms in Abhängigkeit von dem bestimmten Gehalt der Mehrzahl von Begleitelementen eingestellt. Durch die Anpassung des Massenstroms des zugeführten Sauerstoffstroms wird auch die Temperatur in der Brennstoffvergasungsvorrichtung eingestellt. Sind die Gehalte der Begleitelemente bekannt, kann dieser wichtige Betriebsparameter optimal gewählt werden. Der Sauerstoffstrom bildet in dem Brennstoffvergasungsverfahren gewöhnlich einen Teil des Vergasungsmittels. Der Massenstrom des Sauerstoffstroms kann daher beispielsweise durch Ändern des Massenstroms des zugeführten Vergasungsmittels und/oder durch Ändern der Zusammensetzung des Vergasungsmittels eingestellt werden.

Ein zweiter Erfindungsaspekt betrifft eine Brennstoffvergasungsvorrichtung mit einer Analysevorrichtung, die ausgebildet ist, einen Gehalt einer Mehrzahl von Begleitelementen in einem der Brennstoffvergasungsvorrichtung zugeführten Brennstoff zu bestimmen. Dabei ist die Brennstoffvergasungsvorrichtung dazu ausgebildet, das erfindungsgemäße Verfahren auszuführen.

Dabei ist die Analysevorrichtung vorzugsweise dazu ausgebildet, ein Röntgenfluoreszenzanalyseverfahren, ein Laserinduced-Breakdown-Spektroskopie-Verfahren oder ein Prompt-Gamma-Neutron-Activation-Analysis-Verfahren durchzuführen. Diese automatisierbaren Verfahren sind für die Bestimmung von Zusammensetzungen von Stoffen als zuverlässig und genau bekannt.

Ein weiterer Aspekt der Erfindung betrifft die Verwendung einer erfindungsgemäßen Brennstoffvergasungsvorrichtung für die Herstellung eines Synthesegases.

### Kurzbeschreibung der Abbildungen

Die Erfindung wird nachfolgend anhand von Abbildungen näher erläutert. Es zeigen:
- Figur 1: ein erstes Ausführungsbeispiel einer erfindungsgemäßen Brennstoffvergasungsvorrichtung;
- Figur 2: ein zweites Ausführungsbeispiel einer erfindungsgemäßen Brennstoffvergasungsvorrichtung;
- Figur 3: ein Phasendiagramm des Systems CaO-Al₂O₃-SiO₂.

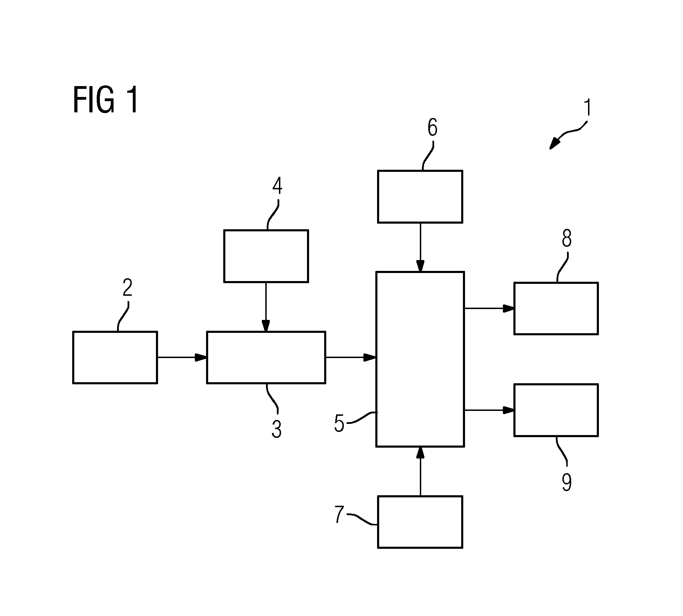

Figur 1 zeigt ein erstes Ausführungsbeispiel einer erfindungsgemäßen Brennstoffvergasungsvorrichtung 1. Aus einem Brennstofflager 2, in dem beispielsweise Kohle gelagert sein kann, wird über eine Fördervorrichtung 3 Brennstoff einem Brennstoffvergasungsreaktor 5 zugeführt. Der Brennstoffvergasungsreaktor 5 kann nach einem der bekannten Verfahren zur Brennstoffvergasung arbeiten, beispielsweise nach der Flugstromvergasung. Der dem Brennstoffvergasungsreaktor 5 zugeführte Brennstoff wird dabei durch eine Analysevorrichtung 4 eine Untersuchung unterzogen, die Aufschluss über die elementare Zusammensetzung des Brennstoffs und somit über einen Gehalt an Begleitelementen gibt. Die Analysevorrichtung 4 kann beispielsweise ausgebildet sein, ein Röntgenfluoreszenzanalyseverfahren, ein Laser-induced-Breakdown-Spektroskopie-Verfahren oder ein Prompt-Gamma-Neutron-Activation-Analysis-Verfahren durchzuführen.

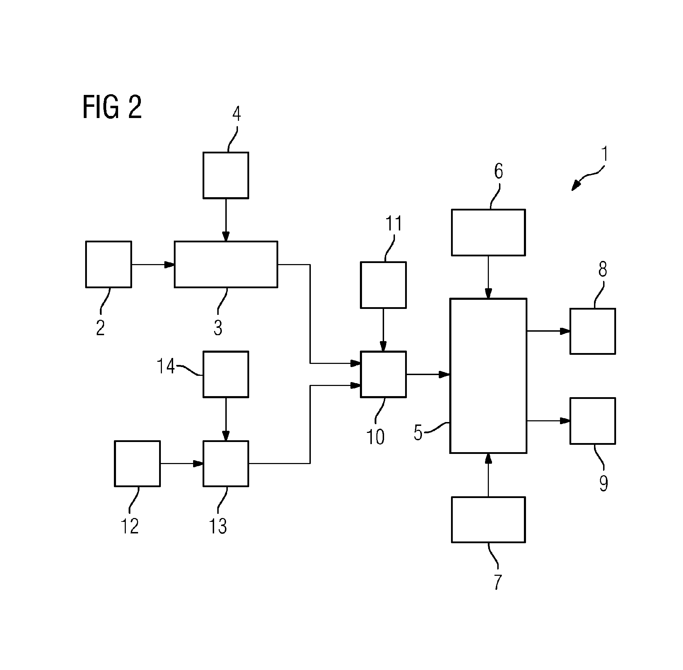

Die Untersuchung kann auf eine bestimmte Auswahl an Begleitelementen beschränkt sein, um die Untersuchung zu beschleunigen. Die Untersuchung kann entweder an der Gesamtheit des dem Brennstoffvergasungsreaktor 5 zugeführten Brennstoffs oder an dem Brennstoff entnommenen Proben durchgeführt werden. Vorzugsweise wird die Untersuchung kontinuierlich oder periodisch durchgeführt. Zusätzliche Untersuchungen können vorgesehen werden, wenn durch Zuführen einer anderen Charge Brennstoff oder Wechsel des Brennstofftyps eine starke Änderung der Zusammensetzung des Brennstoffs zu erwarten ist. Ein Sauerstoffgenerator 6 führt dem Brennstoffvergasungsreaktor 5 gasförmigen Sauerstoff zu. Der Sauerstoffgenerator 6 ist vorzugsweise ausgebildet, nach im Stand der Technik bekannter Art reinen Sauerstoff zu erzeugen, um eine Verunreinigung des Vergasungsprozesses durch andere Gase wie Stickstoff zu vermeiden. Der zugeführte gasförmige Sauerstoff verbrennt einen Teil des in dem Brennstoffvergasungsreaktor 5 enthaltenen Brennstoffs, wodurch die für den Vergasungsprozess benötigten Temperaturen in dem Brennstoffvergasungsreaktor 5 erzeugt werden. Die Temperatur kann dabei über die Menge des zugeführten Sauerstoffstroms gesteuert werden. Eine Wasserzuführung 7 führt dem Brennstoffvergasungsreaktor 5 Wasser zu, das mit dem im Brennstoff enthaltenen Kohlenstoff in einer endothermen Reaktion vorwiegend zu Kohlenmonoxid und Wasserstoff reagiert, die die wesentlichen Bestandteile des Synthesegases bilden. Das Synthesegas kann durch eine Filter- und Sauergaswäschevorrichtung 8 geleitet werden, wo im Synthesegas enthaltene feine Aschepartikel aus dem Synthesegas herausgefiltert sowie Kohlendioxid, Schwefelverbindungen wie H₂S und COS und Spurenverbindungen ausgewaschen werden. Eine Abraumvorrichtung 9 kann vorgesehen sein, um überschüssige grobe Schlacke aus dem Brennstoffvergasungsreaktor 5 abzuführen. Figur 2 zeigt ein zweites Ausführungsbeispiel einer erfindungsgemäßen Brennstoffvergasungsvorrichtung 1. Das zweite Ausführungsbeispiel der Erfindung entspricht im Wesentlichen dem ersten Ausführungsbeispiel, so dass das zum ersten Ausführungsbeispiel Gesagte auch für das zweite gilt, sofern nichts Abweichendes beschrieben wird.

Das zweite Ausführungsbeispiel der Erfindung verfügt über ein zweites Brennstofflager 12, aus dem über eine zweite Fördervorrichtung 13 dem Brennstoffvergasungsreaktor 5 ein alternativer Brennstoff zugeführt werden kann. Der alternative Brennstoff wird dabei durch eine zweite Analysevorrichtung 14 untersucht. Das für das erste Brennstofflager 2, die erste Fördervorrichtung 3 und die erste Analysevorrichtung 4 Gesagte gilt hier entsprechend. Die beiden Brennstoffe können einer Mischvorrichtung 10 zugeführt werden, die die Brennstoffe in einem wählbaren Mischungsverhältnis vermischt und die so vermischten Brennstoffe dem Brennstoffvergasungsreaktor 5 zuführt. Das Mischungsverhältnis wird dabei in Abhängigkeit der Untersuchungsergebnisse der Analysevorrichtungen 4 und 14 gewählt, um gewünschte Gehalte an Begleitelementen in dem resultierenden gemischten Brennstoff zu erhalten.

Es kann eine Begleitelementenzuführvorrichtung 11 vorgesehen sein, die dazu ausgebildet ist, in Abhängigkeit von den von den Analysevorrichtungen 4 und 14 festgestellten Gehalten an Begleitelementen in den Brennstoffen dem dem Brennstoffvergasungsreaktor 5 zugeführten Brennstoff Begleitelemente beizumischen. Dies kann beispielsweise durch Hinzufügung gängiger Verbindungen der gewünschten Begleitelemente geschehen, beispielsweise von Kalkstein/Calciumcarbonat (Lieferant von CaO), Tonerden oder Bauxiten (Lieferant von Al₂O₃) und/oder Quarzsand Lieferant von SiO₂). Es können auch andere Substanzen beigemischt werden, die schlackebildenden Elemente in bekannter Zusammensetzung enthalten. Fügt man zum Beispiel Tonerden oder Ziegelmehl zu, so kommt, entsprechend der chemischen Zusammensetzung, ein Alumosilikat dazu, das den Gehalt von Silicium und Aluminium gleichermaßen erhöht. Die Gehalte der Begleitelemente können dabei beispielsweise so eingestellt werden, dass eine möglichst niedrige Schmelztemperatur der bei der Brennstoffvergasung entstehenden Asche oder ein vorteilhaftes Fließverhalten der geschmolzenen flüssigen Schlacke erreicht wird, um die oben erläuterten Vorteile zu erreichen. Eine solche Begleitelementenzuführvorrichtung 11 kann auch bei einer Brennstoffvergasungsvorrichtung 1 mit nur einem Brennstofflager 2, einer Fördervorrichtung 3 und einer Analysevorrichtung 4, wie sie in Figur 1 gezeigt wird, vorgesehen sein.

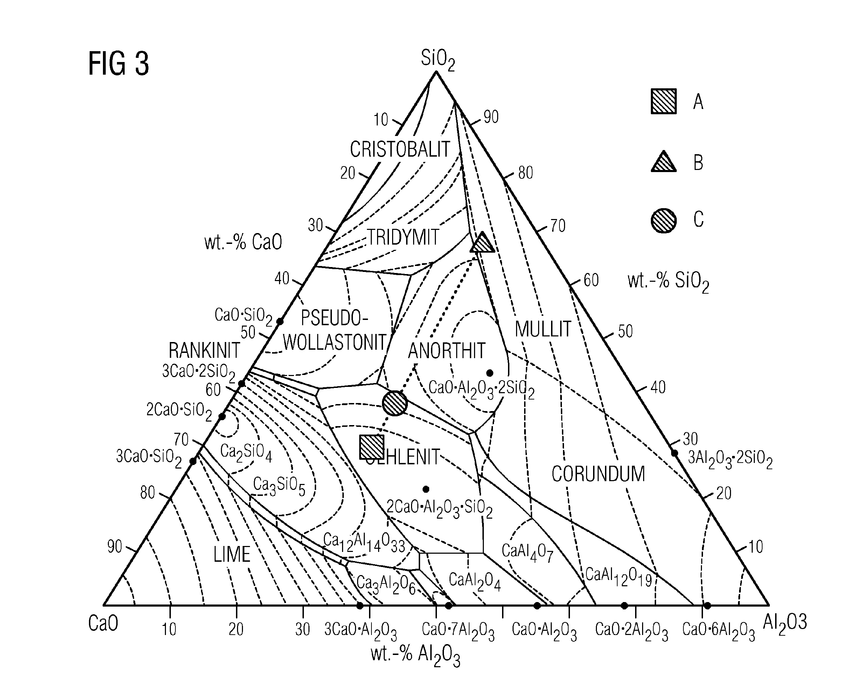

Figur 3 zeigt ein Phasendiagramm des Systems CaO-Al₂O₃-SiO₂. Das Phasendiagramm zeigt Schmelztemperaturen für Gemische der Bestandteile Calciumoxid (CaO), Aluminiumoxid (Al₂O₃) und Siliciumdioxid (SiO₂), die die typischen Hauptbestandteile der in Brennstoffvergasungsprozessen entstehenden Asche darstellen. Das Mischungsverhältnis der drei Bestandteile ergibt sich im Diagramm aus der Position eines gewählten Punktes relativ zu den Ecken des Diagramms. In den Ecken des dreieckförmigen Diagramms enthalten die Gemische 100 Gewichtsprozente des der jeweiligen Ecke zugeordneten Stoffs, der Mittelpunkt des Diagramms bedeutet eine Mischung der drei Bestandteile zu gleichen Teilen. Das Diagramm enthält neben Abgrenzungen unterschiedlicher Minerale wie Mullit, Anorthit etc., Isothermen, die den Schmelzpunkt eines jeweiligen Mischungsverhältnisses der drei Bestandteile anzeigen.

In dem Phasendiagramm sind drei Punkte A, B und C eingetragen, die Mischungsverhältnis der Stoffe Calciumoxid, Aluminiumoxid und Siliciumdioxid für einen jeweiligen Brennstoff beziehungsweise ein Brennstoffgemisch angeben. Dies bedeutet, dass Asche mit dem bezeichneten Mischungsverhältnis der drei Bestandteile verbleibt, wenn der in dem Brennstoff enthaltene Kohlenstoff in das Synthesegas überführt ist. Der Punkt A (quadratische Markierung) kann die Gehalte für einen ersten Brennstoff bezeichnen, beispielsweise eine preiswerte Kohle, deren Asche einen Schmelzpunkt von über 1500 Grad Celsius aufweist. Punkt B bezeichnet die entsprechenden Gehalte für einen zweiten Brennstoff, beispielsweise eine etwas teurere Kohle aus einer anderen Förderstätte, für die die Asche ebenfalls einen Schmelzpunkt von über 1500 Grad Celsius aufweist. Durch Mischung der beiden Brennstoffe in einem wählbaren Verhältnis kann ein Punkt C auf einer Verbindungslinie zwischen den beiden Punkten A und B gewählt werden, der ein bestimmtes Mischungsverhältnis der beiden Brennstoffe bedeutet. Die Verbindungslinie ist in Figur 3 als gerade Strecke aufgetragen, je nach Darstellung des Phasendiagramms kann sich jedoch auch eine Kurve ergeben. Die Endpunkte dieser Verbindungslinie werden durch ein Mischungsverhältnis von 100 Prozent des jeweiligen Brennstoffs und 0 Prozent des anderen Brennstoffs gebildet, jeder Punkt auf der Verbindungslinie entspricht einem bestimmten Mischungsverhältnis. Das Mischungsverhältnis der beiden Brennstoffe wird vorzugsweise so gewählt, dass ein möglichst niedriger Schmelzpunkt der Asche des Brennstoffgemischs erreicht wird und die geschmolzene Asche bestimmte Fließeigenschaften aufweist. Dabei können allerdings auch die unterschiedlichen Kosten für die Brennstoffe berücksichtigt werden, die unter Umständen die Kosten für einen erhöhten Sauerstoffverbrauch bei der Brennstoffvergasung kompensieren. So ist in dem Phasendiagramm ein Punkt auf der Verbindungslinie auffindbar, der nahe dem Punkt B liegt und eine ähnlich niedrige Schmelztemperatur wie Punkt C aufweist, dafür aber einen wesentlich höheren Anteil der teureren zweiten Kohle des Beispiels bedingt. Das dem Punkt C zugeordnete Mischungsverhältnis weist hingegen einen wesentlich höheren Anteil der preiswerteren Kohle auf, weshalb dieses Mischungsverhältnis auch bei einer etwas höheren Schmelztemperatur der entstehenden Asche bevorzugt werden kann.

Es können auch mehr als zwei verschiedene Brennstoffe vermischt werden. Dies würde in dem Phasendiagramm zu einer Fläche führen, innerhalb derer ein bevorzugtes Mischungsverhältnis der mehr als zwei Brennstoffe gewählt werden kann (bei mehr als drei Brennstoffen können auch mehrere Mischungsverhältnisse einem Punkt in dem Phasendiagramm zugeordnet sein). Außerdem können zur Erhöhung der Freiheitsgrade bei der Auswahl eines Punktes in dem Phasendiagramm chemische Verbindungen zur gezielten Veränderung der Gehalte an Calciumoxid, Aluminiumoxid und/oder Siliciumdioxid dem Brennstoff in der benötigten Menge beigemengt werden. Dies kann auch dann geschehen, wenn nur ein Brennstoff für die Brennstoffvergasung verwendet wird.

Das zur Figur 3 Gesagte kann selbstverständlich auch auf andere typische Bestandteile von Asche und auf mehr als drei Bestandteile ausgedehnt werden.

Obwohl die Erfindung im Detail durch Ausführungsbeispiele von bevorzugten Ausführungsformen näher illustriert und beschrieben wurde, ist die Erfindung nicht durch die offenbarten Beispiele eingeschränkt. Variationen der Erfindung können vom Fachmann aus den gezeigten Ausführungsbeispielen abgeleitet werden, ohne den Schutzumfang der Erfindung, wie er in den Ansprüchen definiert wird, zu verlassen.

## Patentansprüche

1. Ein Verfahren zum Betreiben einer
Brennstoffvergasungsvorrichtung (1) mit den Schritten:
Zuführen von kohlenstoffbasiertem Brennstoff zu der Brennstoffvergasungsvorrichtung (1);
Zuführen eines Vergasungsmittels zu der Brennstoffvergasungsvorrichtung (1);
Erhitzen des Brennstoffs und des Vergasungsmittels in der Brennstoffvergasungsvorrichtung (1) in einer sauerstoffarmen Atmosphäre für die Produktion eines Synthesegases,
**dadurch gekennzeichnet, dass** der zugeführte Brennstoff von einer Analysevorrichtung (4, 14) untersucht und dabei ein jeweiliger Gehalt einer Mehrzahl von Begleitelementen an dem Brennstoff bestimmt wird,
wobei die Brennstoffvergasungsvorrichtung (1) in Abhängigkeit von den bestimmten Gehalten der Mehrzahl von Begleitelementen betrieben wird.

2. Das Verfahren des vorhergehenden Anspruchs,
bei dem die Zuführung des Brennstoffs gestoppt wird, wenn eine Überschreitung eines Grenzwertes durch den Gehalt eines bestimmten Begleitelementes durch die Analysevorrichtung (4, 14) festgestellt wird.

3. Das Verfahren von einem der beiden vorhergehenden Ansprüche,
bei dem die von der Analysevorrichtung (4, 14) bestimmten jeweiligen Gehalte der Begleitelemente für einen Betriebszyklus der Brennstoffvergasungsvorrichtung (1) akkumuliert werden und die Brennstoffvergasungsvorrichtung (1) in Abhängigkeit der akkumulierten bestimmten Gehalte der Begleitelemente betrieben wird.

4. Das Verfahren des vorhergehenden Anspruchs,
bei dem der Betriebszyklus der Brennstoffvergasungsvorrichtung (1) beendet wird, wenn einer oder mehrere der akkumulierten bestimmten Gehalte einen Schwellwert überschreitet.

5. Das Verfahren des vorhergehenden Anspruchs,
bei dem nach Beendigung des Betriebszyklusses eine Wartung der Brennstoffvergasungsvorrichtung (1), eine Reinigung der Brennstoffvergasungsvorrichtung (1) oder ein Austausch eines Katalysators der Brennstoffvergasungsvorrichtung (1) durchgeführt werden.

6. Das Verfahren eines der vorhergehenden Ansprüche,
bei denen wenigstens ein resultierender Gehalt der Mehrzahl von Begleitelementen angepasst und dadurch ein Schmelzpunkt einer während des Betreibens der Brennstoffvergasungsvorrichtung (1) entstehenden Asche geändert wird.

7. Das Verfahren des vorhergehenden Anspruchs,
bei dem die resultierenden Gehalte der Begleitelemente angepasst werden, indem das Zuführen des Brennstoffs ein Zuführen eines ersten Ausgangsbrennstoffs und eines von dem ersten Ausgangsbrennstoff verschiedenen zweiten Ausgangsbrennstoffs umfasst, indem das Bestimmen der Gehalte der Begleitelemente jeweils für den ersten Ausgangsbrennstoff und für den zweiten Ausgangsbrennstoff durchgeführt wird und indem ein Mischungsverhältnis von erstem und zweitem Ausgangsbrennstoff in Abhängigkeit der bestimmten Gehalte der Mehrzahl von Begleitelemente gewählt wird.

8. Das Verfahren von einem der beiden vorhergehenden Ansprüche,
mit einem zusätzlichen Schritt des Zuführens von Zusatzstoffen zu der Brennstoffvergasungsvorrichtung in Abhängigkeit von den bestimmten Gehalten der Begleitelemente.

9. Das Verfahren von einem der drei vorhergehenden Ansprüche,
bei dem resultierende Gehalte von Calciumoxid, Aluminiumoxid und Siliziumdioxid angepasst werden.

10. Das Verfahren von einem der vorhergehenden Ansprüche, bei dem ein Massenstrom eines zu der Brennstoffvergasungsvorrichtung (1) zugeführten Sauerstoffstroms in Abhängigkeit von dem bestimmten Gehalt der Mehrzahl von Begleitelementen eingestellt wird.

11. Eine Brennstoffvergasungsvorrichtung (1) mit einer Analysevorrichtung (4, 14), die ausgebildet ist, einen Gehalt einer Mehrzahl von Begleitelementen in einem der Brennstoffvergasungsvorrichtung (1) zugeführten Brennstoff zu bestimmen,
**dadurch gekennzeichnet, dass**
die Brennstoffvergasungsvorrichtung (1) dazu ausgebildet ist, das Verfahren von einem der vorhergehenden Ansprüche auszuführen.

12. Die Brennstoffvergasungsvorrichtung (1) des vorhergehenden Anspruchs,
der die Analysevorrichtung (4, 14) ausgebildet ist, ein Röntgenfluoreszenzanalyseverfahren, ein Laser-induced-Breakdown-Spektroskopie-Verfahren oder ein Prompt-Gamma-Neutron-Activation-Analysis-Verfahren durchzuführen.

13. Verwendung einer Brennstoffvergasungsvorrichtung (1) gemäß einem der beiden vorhergehenden Ansprüche für die Herstellung eines Synthesegases.
